(19) **Europäisches Patentamt**

**European Patent Office**

**Office européen des brevets**

(11) Veröffentlichungsnummer: **0 418 625 A1**

(12) **EUROPÄISCHE PATENTANMELDUNG**

(21) Anmeldenummer: 90116858.3

(22) Anmeldetag: 03.09.90

(51) Int. Cl.5: **C12N 15/57**, C12N 1/21, //(C12N1/21,C12R1:07), (C12N1/21,C12R1:19)

(30) Priorität: 06.09.89 DE 3929531

(43) Veröffentlichungstag der Anmeldung: 27.03.91 Patentblatt 91/13

(84) Benannte Vertragsstaaten: BE CH DE DK ES FR GB IT LI NL

(71) Anmelder: **HOECHST AKTIENGESELLSCHAFT** **Postfach 80 03 20** **W-6230 Frankfurt am Main 80(DE)**

(72) Erfinder: **Marquardt, Rüdiger, Dr.** **Günthersburgallee 69** **W-6000 Frankfurt am Main(DE)** Erfinder: **Hilgenfeld, Rolf, Dr.** **Kleegartenstrasse 46** **W-6382 Friedrichsdorf(DE)** Erfinder: **Keller, Reinhold, Dr.** **Alleestrasse 18** **W-6232 Bad Soden(DE)**

(54) **Proteasegen aus Bacillus thermoproteolyticus rokko, Verfahren zu seiner Gewinnung und seine Verwendung.**

(57) Das Thermolysin-Gen aus Bacillus thermoproteolyticus rokko kann gewonnen werden, indem Gesamt-DNA aus Bacillus thermoproteolyticus rokko isoliert wird, diese mit Restriktionsendonukleasen in Fragmente unterschiedlicher Größe zerteilt wird, die Fragmente durch Hybridisierung mit Oligonukleotiden identifiziert werden und isoliert werden.

EP 0 418 625 A1

## PROTEASEGEN AUS BACILLUS THERMOPROTEOLYTICUS ROKKO, VERFAHREN ZU SEINER GEWINNUNG UND SEINE VERWENDUNG.

Die vorliegende Erfindung betrifft die Klonierung eines Thermolysin Gens aus Bacillus thermoproteolyticus rokko, den Transfer dieses Gens in E.coli sowie in Bacillus und die Herstellung des Genprodukts.

Die neutrale Protease Thermolysin aus dem Stamm Bacillus thermoproteolyticus rokko besteht aus einer einzelnen Polypeptid-Kette mit 316 Aminosäuren und benötigt $Zn^{2+}$ für die katalytische Aktivität sowie vier Calcium-Ionen für die ausgeprägte thermische Stabilität (Fontana, A.: Biophysical Chemistry 29 181-193 (1988)). Die vollständige Aminosäure-Sequenz dieses Proteins wurde ebenfalls veröffentlicht (Titani et al.: Nature 238 35-37 (1972)).

Proteasen mit unterschiedlich stark auspeprägter Ähnlichkeit zu dem aus Bacillus thermoproteolyticus erhältlichen Enzym werden von einer Reihe anderer Bacillus species produziert, u.a. Bacillus stearothermophilus, Bacillus amyloliquefaciens, Bacillus subtilis und Bacillus cereus. Die für die entsprechenden Enzyme codierenden Gene sind beispielsweise aus B.stearothermophilus, B.subtilis und B.amyloliquefaciens kloniert und sequenziert worden (Takagi et al.: J. Bact. 163 824-831 (1985); Yang, M.Y. et al.: J. Bact. 160 , 15-21 (1984)); Vasantha et al.: J. Bact. 159 811-819 (1984)).

Es wurde nun das Gen für die neutrale Protease aus Bacillus thermoproteolyticus rokko gefunden. Die Erfindung betrifft somit das Gen für die neutrale Protease aus Bacillus thermoproteolyticus rokko und die dazugehörige Präprosequenz, die aus 232 Aminosäuren besteht. Das Gen mit seiner Präprosequenz besitzt die in Tabelle 1 angegebene DNA-sequenz. Die Präprosequenz beginnt mit der Position 328 und endet bei 1023. Der für das native Thermolysin kodierende Anteil des Gens beginnt mit der Position 1024 und endet mit der Position 1974. Innerhalb der DNA-Sequenz des Gens für das native Thermolysin ist der sehr geringe Gehalt von G+C in der dritten Position der jeweiligen Tripletts besonders erwähnenswert. Überraschenderweise weicht die aus der DNA-Sequenz ableitbare Aminosäure-Sequenz für das fertige, ausgeschleuste Thermolysin-Enzym an zwei Stellen von der bislang angenommenen Sequenz ab, nämlich 37Asn anstelle von Asp und 119Gln anstelle von Glu.

Das erfindungsgemäße Gen für die neutrale Protease aus Bacillus thermoproteolyticus rokko kann wie folgt isoliert werden:

Die Gesamt-DNA aus Bacillus thermoproteolyticus rokko wird nach gängigen Methoden isoliert und mit verschiedenen Restriktionsendonukleasen, z.B. Dral, Clal, Hpal und HindIII in Fragmente unterschiedlicher Größe zerteilt, die dann durch Agarose-Gelelektrophorese aufgetrennt werden. Nach Transfer dieser Fragmente auf Filter wird mit radioaktiv markierten Oligonukleotid-Sonden hybridisiert, die entsprechend der bekannten Aminosäure-Sequenz des Thermolysin chemisch synthetisiert werden können. Besonders eignen sich für diese Vorgehensweise Oligonukleotide der folgenden Sequenz, wobei sich die Zahlen über der Aminosäuresequenz auf die Numerierung des prozessierten Thermolysin beziehen.

```
          55                    60
 -Trp-Ala-Asp-Ala-Asp-Asn-Gln-Phe-Phe-Ala-
 -TGG-GCA-GAT-GCA-GAT-AAC-CAA-TTT-TTT-GC
          T    C    T    C    T         C    C


          110                   115
 -Gln-Gly-Tyr-Asn-Asn-Ala-Phe-Trp-Asn-Gly-
 -CAA-GGA-TAT-AAT-AAC-GCA-TTT-TGG-AAC-GG
          T    C    C    T    T    C         T
```

Es handelt sich um gemischte Oligonukleotide, bei denen an den jeweils bezeichneten Stellen beide möglichen Nukleotide verwendet worden sind. Es resultiert in beiden Fällen eine 128fache Degeneration der jeweils aus 29 Nukleotiden bestehenden Sonden.

Nach vollständiger Verdauung von Bacillus thermoproteolyticus rokko DNA mit den obengenannten Restriktionsenzymen in Einzelansätzen können Fragmente, die durch Hybridisierung identifiziert wurden, mittels Elektroelution aus einem Agarose-Gel isoliert und mit Plasmid-Vektoren verbunden werden. Die Ligase-Ansätze werden in geeignete Wirtsstämme transformiert, Klone selektioniert und aus diesen Klonen die gewünschten Fragmente identifiziert.

Durch direkte Klonierung der Fragmente in Sequenziervektoren, z.B. in pUC8 oder die daraus abgeleiteten Vektoren der pSVB-Reihe (Arnold und Pühler, Gene 70 171-179 (1988)), können die Fragmente sequenziert werden. Mit Hilfe weiterer Subklonierungen oder gezielter Deletionen innerhalb der klonierten Fragmente kann der gesamte Bereich eines klonierten Fragments erfaßt werden. Das Strukturgen sowie die zusätzlichen 5'- und 3'-Bereiche können durch Computer-Programme eindeutig identifiziert werden.

DNA-Fragmente, auf denen das Thermolysin-Gen vollständig enthalten ist, werden zur Expression in Bacillus Vektoren oder in Bacillus Shuttle-Vektoren, z.B. pRB273 (Brückner et al., Gene 32 , 151-60 (1984)) und pUB110 (Gryczan et al., 134 , 318 (1978)) kloniert. Nach Transformation mit diesen Vektoren kann das prozessierte Thermolysin-Protein nach Wachstum der Stämme in geeigneten Medien aus diesen isoliert werden.

Überraschend wurde nun gefunden, daß die Expression von Thermolysin in E. coli ebenfalls möglich ist, wobei das prozessierte Enzym in der Nährlösung von E. coli gefunden werden kann.

Zur Expression des Thermolysin-Gens in E. coli wird ein DNA-Fragment, das das Gen für das native Thermolysin sowie für dessen Präprosequenz enthält, mit einem Vektor, z.B. pBR 322 oder den von pUC abgeleiteten Vektoren pSVB26 oder pSVB23, verbunden und in E. coli K12 Stämme, wie HB 101, MM294 oder DH1, transformiert. Es können auch noch Promotoren vor das Gen geschaltet werden. Beispielhaft sollen hier lac-, tac- oder RP$_2$-Promotoren genannt werden. Es können natürlich auch alle anderen in E. coli anwendbaren Promotoren eingesetzt werden.

Die im folgenden aufgeführten Beispiele sollen die Erfindung des weiteren erläutern. Prozentangaben beziehen sich auf das Gewicht, sofern nichts anderes angegeben ist.

Beispiel 1

Lokalisierung der das Thermolysingen tragenden DNA-Fragmente

Gesamt-DNA aus Bacillus thermoproteolyticus rokko wurde isoliert und mit den Restriktionsenzymen DraI, ClaI, HpaI und HindIII verdaut und mit Gelelektrophorese aufgetrennt.

Die Fragmente wurden durch Southern Blotting auf Filter transferiert und gegen radioaktiv markierte chemisch synthetisierte Oligonukleotide (Phosphoramiditmethode, Beaucage and Caruthers, Tetrahedron Letters 22 , 1859 (1981)) mit der folgenden Sequenz hybridisiert und anschließend autoradiographiert.

```
           55                        60
  -Trp-Ala-Asp-Ala-Asp-Asn-Gln-Phe-Phe-Ala-
  -TGG-GCA-GAT-GCA-GAT-AAC-CAA-TTT-TTT-GC
        T    C    T    C    T         C    C


          110                       115
  -Gln-Gly-Tyr-Asn-Asn-Ala-Phe-Trp-Asn-Gly-
  -CAA-GGA-TAT-AAT-AAC-GCA-TTT-TGG-AAC-GG
        T    C    C    T    T    C         T
```

Den sichtbar gemachten Fragmenten konnten folgende Größen zugeordnet werden:

DraI: 1,2kb
HpaI: 2,4kb
HindIII: 3,7kb
ClaI: 4,8kb

Die Fragmente werden in das Plasmid pSVB 23 oder 26 kloniert und vollständig oder teilweise sequenziert. Auf dem ClaI-Fragment konnte das vollständige Gen, i.e. für das native Thermolysin sowie für dessen Präprosequenz, gefunden werden.

Beispiel 2

Hybridisierung von filtergebundener DNA

Die Hybridisierung wurde im wesentlichen so durchgeführt, wie sie in dem von der Firma Gibco BRL herausgegebenen Heft "focus", Vol.9 Nr.2, 1987, auf den Seiten 1-2 beschrieben ist.

Das Filter wurde mindestens 2h mit Vorhybridisierungs-Puffer bei 65°C gewaschen (5xSSC, 20mM Natriumphosphat (pH 7,0), 10xDenhardts [1 g Ficoll (nicht-ionisches, synthetisches Saccharose-Polymer, MG 4000), 1 g Polyvinylpyrrolidon, 1 g Rinderserumalbumin in 500 ml $H_2O$], 7% SDS, 100 $\mu$g/ml denaturierte Lachssperm-DNA - die Lachssperm-DNA war durch 15minutiges Kochen in Wasser denaturiert worden). Dann wurde vorgewärmte Dextran-Sulfat Lösung (50%) bis zu einer Endkonzentration von 10% zugefügt und radioaktiv markiertes Oligonukleotid bis zu einer Konzentration von 1-5ng/ml Hybridisierungslösung zugegeben. Anschließend wurde mindestens 16h im Schüttelwasserbad unter leichtem Schütteln inkubiert.

Die Hybridisierungs- und anschließend die Waschtemperatur lag zwischen 50-55°C.

Nach erfolgter Inkubation wurde die Hybridisierungslösung abgegossen und konnte noch in weiteren Hybridisierungsexperimenten eingesetzt werden. Vom Filter wurden Reste an Hybridisierungslösung möglichst vollständig abgestreift und dann auf die Hybridisierungstemperatur vorgewärmter Waschpuffer I (3xSSC, 10mM Natriumphosphat pH 7,0, 10xDenhardts, 5%SDS) zugegeben. Nach einer Stunde Schütteln bei der Hybridisierungstemperatur wurde der Waschpuffer I durch Waschpuffer II ersetzt (1xSSC, 1%SDS) und dieser nach 30min gewechselt.

Nach 30min in frischem Waschpuffer II wurde der feuchte Filter in Frischhaltefolie eingewickelt und in einer Kassette auf röntgenempfindlichem Film exponiert (z.B. Kodak, XS1). Die Expositionszeiten betrugen bei -80°C zwischen 4h und 5 Tagen. War der Hintergrund an unspezifischer Strahlung zu hoch, so konnten die Filter nochmals in Waschpuffer II bei einer gegenüber der Hybridisierung erhöhten Temperatur für 1 Stunde nachgewaschen werden. Anschließend erfolgte erneute Autoradiographie.

Beispiel 3

Koloniehybridisierung zur Identifizierung

Gewachsene Bakterienkolonien auf Agarplatten wurden einige Stunden im Kühlschrank bei 4°C gekühlt. Dann wurde ein Plaque/Colony Screen Filter (Colony/Plaque ScreenTM, NEN Research Products, Katalog Nr. NEF-978/978A), der im Durchmesser etwas kleiner war als die Agarplatte, aufgelegt und die Lage des Filters mit Kugelschreiber und Nadelstichen markiert. Das Filter wurde nach 2-3min abgezogen und mit den anhaftenden Bakterienkolonien nach oben zunächst 5min auf ein mit 10%igem SDS getränktes Whatman 3MM Papier gelegt. Anschließend wurde das Filter 5min auf Whatman 3MM Papier transferiert, das zuvor in 0,5M NaOH, 1,5M NaCl getränkt worden war. Ein erneuter Transfer für 5min auf Whatman 3MM Papier, das zuvor in 0,5M Tris-Cl pH 8,0, 1,5M NaCl getränkt worden war, wurde von einem letzten Transfer auf trockenes Whatman 3MM Papier gefolgt. Nach Trocknen wurde das Filter mehrere Stunden in Puffer (3xSSC, 0,1%SDS) bei 65°C geschüttelt, um anhaftende Koloniereste zu entfernen. Der Puffer wurde mehrmals gewechselt. Das Filter stand anschließend für Hybridisierungen zur Verfügung.

Beispiel 4

Transformation von Bacillus

Zur Herstellung kompetenter Zellen wurde eine Übernacht-Kultur von B.subtilis in HS-Medium [66,5ml aqua bidest, 10ml 10xS-Base, 2,5ml 20% Glukose, 5ml D,L-Tryptophan (1mg/ml), 1ml 2% Caseinhydrolysat, 5ml 2% Hefeextrakt, 10ml Aminosäure-Mischung (8%Arginin, 0,4% Histidin); alle Komponenten getrennt autoklaviert oder sterilfiltriert und anschließend zusammengegeben] kultiviert. 10xS-Base (Spizizens Salz): 2g (NH4)$_2$SO4, 14g K$_2$HPO4, 6g KH$_2$PO4, 1g Na-citrat x 2H$_2$O werden mit aqua bidest auf 100ml aufgefüllt; nach dem Autoklavieren werden 0,1ml einer sterilfiltrierten 1M MgSO$_4$-Lösung zugegeben und dergestalt mit 50ml frischem HS-Medium verdünnt, daß eine Ausgangs OD$_{578}$ von 0,1 resultierte. Die Zellen wurden bei 37°C inkubiert und durch Vergleich mit einer zuvor erstellten Wachstumskurve der Übergang in die stationäre Phase festgestellt. Dieser Punkt war unter den gewählten Bedingungen nach rund 4-5 Stunden erreicht. Zu diesem Zeitpunkt wurden 5ml der Suspension entnommen, mit 0,5ml sterilem Glycerin gut durchmischt und in Aliquots von 0,5ml oder 1ml bei -85°C eingefroren. Alternativ können die Zellen auch ohne Zugabe von Glycerin direkt in der nachfolgend beschriebenen Transformation eingesetzt werden.

Zur Transformation wurden 1ml der eingefrorenen Zellen bei 37°C aufgetaut und in 20ml LS-Medium (80ml aqua bidest, 10ml 10xS-Base, 2,5ml 20% Glukose, 0,5ml D,L-Tryptophan (1mg/ml), 0,5ml 2% Casein-Hydrolysat, 5ml 2% Hefeextrakt, 0,25ml 1M MgCl$_2$, 0,05ml 1M CaCl$_2$) bei 30°C 2 Stunden inkubiert. 1ml dieser Inkubations-Mischung wurden sodann in ein 10ml Plastikröhrchen (Firma Greiner) gefüllt und mit 10μl einer 0,1M EGTA-Lösung versetzt. Nach 5 Minuten Inkubation bei Raumtemperatur wurde die zu transformierende Plasmid-DNA, i.e. das 4,8 kb ClaI Fragment in dem Vektor pR273, zugegeben (0,5-5μg) und bei 37°C weitere 2 Stunden inkubiert. Anschließend wurden die Zellen abzentrifugiert (10min, 6.000UpM, Heraeus Biofuge) und in rund 100μl des Überstands resuspendiert. Diese aufkonzentrierte Suspension wurde auf LB-Platten (10g Nutrient Broth, 5g NaCl, 5g Hefe-Extrakt) ausplattiert, die 10μg/ml Kanamycin enthielten. Nach Bebrüten über Nacht bei 37°C konnten resistente Kolonien identifiziert werden.

Beispiel 5

Expression in E.coli

Zur Expression des Thermolysin Gens in E.coli wurde das 4,8kb große ClaI-Fragment, auf dem das Thermolysin-Gen vollständig enthalten ist, mit dem Vektor pBR322 verbunden und mittels bekannter Methoden in E. coli K12 Stämme, wie HB101, MM294 und DH1, transformiert. Die von pUC abgeleiteten Vektoren pSVB26 oder 23 konnten ebenfalls verwendet werden. Wurden die entsprechenden Klone auf einer Platte ausgestrichen, die durch Anwesenheit eines Protease-Substrats getrübt war (Calcium-Caseinat-Agar nach FRAZIER und RUPP, modifiziert, Art. Nr. 2248, Firma E.Merck, Darmstadt, FRG), so zeigte sich um die Ausstriche eine deutliche Aufhellung des Agars. Wurden auf L-Broth Agar angewachsene Kolonien ausgestochen und auf einem Agarblöckchen auf den Testagar aufgesetzt, so war um das Blöckchen ebenfalls eine deutliche Aufhellung festzustellen. Die Größe des Lysehofs entsprach dabei ungefähr der Größe, der beim Aufsetzen einer ausgestochenen Kolonie von Bacillus thermoproteolyticus rokko zu beobachten war. Flüssiges L-Broth Medium wurde mit einer Einzelkolonie von Thermolysin-exprimierenden E.coli Bakterien angeimpft und die Bakterien über Nacht geschüttelt. Die Bakterien wurden abzentrifugiert und der Überstand mittels SDS-PAGE getestet. Es zeigte sich, daß im Überstand der E.coli Bakterien ein Protein enthalten war, das in seinem Laufverhalten auf SDS-Gelen identisch war mit hochgereinigtem und käuflich zu erwerbendem Thermolysin aus B.thermoproteolyticus rokko.

Tabelle 1

```
AAGCTTTTATTAGGAATCATGACGTTTGGTATTATGAGTTTACTTGTTCTCATTGGTAGT
        10                    30                    50


GACCAAGAACCAAAATATGTGGCAAAAGACGAACATCCGCCTCCAACCATCATCATTGCA
        70                    90                    110


GCGAAAGATGAACATCCACCAGCAACGATTATTTGAAGAGGAATAAGCAAAAAGACAGCT
        130                   150                   170


AGTTTTCTAGCTGTCTTTTTTCATGCATAGGAAAATGTGAAAAAAACGTAGGGAATTATC
        190                   210                   230


AACTATATCAGACTCTATTTTTCCCAATACAAATACTGTAAATATTGTGTTAATATTCTA
        250                   270                   290


                                    MetLysMetLysMetLysLeuAlaSerPheGly
AATACAAAGAATAAAGGAGGATGAAAAATGAAAATGAAAATGAAATTAGCATCGTTTGGT
        310                   330                   350


LeuAlaAlaGlyLeuAlaAlaGlnValPheLeuProTyrAsnAlaLeuAlaSerThrGlu
CTTGCAGCAGGACTAGCGGCCCAAGTATTTTTACCTTACAATGCGCTGGCTTCAACGGAA
        370                   390                   410


HisValThrTrpAsnGlnGlnPheGlnThrProGlnPheIleSerGlyAspLeuLeuLys
CACGTTACATGGAACCAACAATTTCAAACCCCTCAATTCATCTCCGGTGATCTGCTGAAA
        430                   450                   470


ValAsnGlyThrSerProGluGluLeuValTyrGlnTyrValGluLysAsnGluAsnLys
GTGAATGGCACATCCCCAGAAGAACTCGTCTATCAATATGTTGAAAAAAACGAAAACAAG
        490                   510                   530


PheLysPheHisGluAsnAlaLysAspThrLeuGlnLeuLysGluLysLysAsnAspAsn
TTTAAATTTCATGAAAACGCTAAGGATACTCTACAATTGAAAGAAAAGAAAAATGATAAC
        550                   570                   590
```

```
LeuGlyPheThrPheMetArgPheGlnGlnThrTyrLysGlyIleProValPheGlyAla
CTTGGTTTTACGTTTATGCGCTTCCAACAAACGTATAAAGGGATTCCTGTGTTTGGAGCA
           610                 630                 650


ValValThrAlaHisValLysAspGlyThrLeuThrAlaLeuSerGlyThrLeuIlePro
GTAGTAACTGCGCACGTGAAAGATGGCACGCTGACGGCGCTATCAGGGACACTGATTCCG
           670                 690                 710


AsnLeuAspThrLysGlySerLeuLysSerGlyLysLysLeuSerGluLysGlnAlaArg
AATTTGGACACGAAAGGATCCTTAAAAAGCGGGAAGAAATTGAGTGAGAAACAAGCGCGT
           730                 750                 770


AspIleAlaGluLysAspLeuValAlaAsnValThrLysGluValProGluTyrGluGln
GACATTGCTGAAAAAGATTTAGTGGCAAATGTAACAAAGGAAGTACCGGAATATGAACAG
           790                 810                 830


GlyLysAspThrGluPheValValTyrValAsnGlyAspGluAlaSerLeuAlaTyrVal
GGAAAAGACACCGAGTTTGTTGTTTATGTCAATGGGGACGAGGCTTCTTTAGCGTACGTT
           850                 870                 890


ValAsnLeuAsnPheLeuThrProGluProGlyAsnTrpLeuTyrIleIleAspAlaVal
GTCAATTTAAACTTTTTAACTCCTGAACCAGGAAACTGGCTGTATATCATTGATGCCGTA
           910                 930                 950


AspGlyLysIleLeuAsnLysPheAsnGlnLeuAspAlaAlaLysProGlyAspValLys
GACGGAAAAATTTTAAATAAATTTAACCAACTTGACGCCGCAAAACCAGGTGATGTGAAG
           970                 990                 1010


SerIleThrGlyThrSerThrValGlyValGlyArgGlyValLeuGlyAspGlnLysAsn
TCGATAACAGGAACATCAACTGTCGGAGTGGGAAGAGGAGTACTTGGTGATCAAAAAAAT
           1030                1050                1070


IleAsnThrThrTyrSerThrTyrTyrTyrLeuGlnAspAsnThrArgGlyAsnGlyIle
ATTAATACAACCTACTCTACGTACTACTATTTACAAGATAATACGCGTGGAAATGGGATT
           1090                1110                1130
```

PheThrTyrAspAlaLysTyrArgThrThrLeuProGlySerLeuTrpAlaAspAlaAsp
TTCACGTATGATGCGAAATACCGTACGACATTGCCGGGAAGCTTATGGGCAGATGCAGAT
1150                    1170                    1190

AsnGlnPhePheAlaSerTyrAspAlaProAlaValAspAlaHisTyrTyrAlaGlyVal
AACCAATTTTTTGCGAGCTATGATGCTCCAGCGGTTGATGCTCATTATTACGCTGGTGTG
1210                    1230                    1250

ThrTyrAspTyrTyrLysAsnValHisAsnArgLeuSerTyrAspGlyAsnAsnAlaAla
ACATATGACTACTATAAAAATGTTCATAACCGTCTCAGTTACGACGGAAATAATGCAGCT
1270                    1290                    1310

IleArgSerSerValHisTyrSerGlnGlyTyrAsnAsnAlaPheTrpAsnGlySerGln
ATTAGATCATCCGTTCATTATAGCCAAGGCTATAATAACGCATTTTGGAACGGTTCGCAA
1330                    1350                    1370

MetValTyrGlyAspGlyAspGlyGlnThrPheIleProLeuSerGlyGlyIleAspVal
ATGGTGTATGGCGATGGTGATGGTCAAACATTTATTCCACTTTCTGGTGGTATTGATGTG
1390                    1410                    1430

ValAlaHisGluLeuThrHisAlaValThrAspTyrThrAlaGlyLeuIleTyrGlnAsn
GTCGCACATGAGTTAACGCATGCGGTAACCGATTATACAGCCGGACTCATTTATCAAAAC
1450                    1470                    1490

GluSerGlyAlaIleAsnGluAlaIleSerAspIlePheGlyThrLeuValGluPheTyr
GAATCTGGTGCAATTAATGAGGCAATATCTGATATTTTTGGAACGTTAGTCGAATTTTAC
1510                    1530                    1550

AlaAsnLysAsnProAspTrpGluIleGlyGluAspValTyrThrProGlyIleSerGly
GCTAACAAAAATCCAGATTGGGAAATTGGAGAGGATGTGTATACACCTGGTATTTCAGGG
1570                    1590                    1610

AspSerLeuArgSerMetSerAspProAlaLysTyrGlyAspProAspHisTyrSerLys
GATTCGCTCCGTTCGATGTCCGATCCGGCAAAGTATGGTGATCCAGATCACTATTCAAAG
1630                    1650                    1670

8

```
ArgTyrThrGlyThrGlnAspAsnGlyGlyValHisIleAsnSerGlyIleIleAsnLys
CGCTATACAGGCACGCAAGATAATGGCGGGGTTCATATCAATAGCGGAATTATCAACAAA
        1690              1710              1730


AlaAlaTyrLeuIleSerGlnGlyGlyThrHisTyrGlyValSerValValGlyIleGly
GCCGCTTATTTGATTAGCCAAGGCGGTACGCATTACGGTGTGAGTGTTGTCGGAATCGGA
        1750              1770              1790


ArgAspLysLeuGlyLysIlePheTyrArgAlaLeuThrGlnTyrLeuThrProThrSer
CGCGATAAATTGGGGAAAATTTTCTATCGTGCATTAACGCAATATTTAACACCAACGTCC
        1810              1830              1850


AsnPheSerGlnLeuArgAlaAlaAlaValGlnSerAlaThrAspLeuTyrGlySerThr
AACTTTAGCCAACTTCGTGCTGCCGCTGTTCAATCAGCCACTGACTTGTACGGTTCGACA
        1870              1890            .   1910


SerGlnGluValAlaSerValLysGlnAlaPheAspAlaValGlyValLys
AGCCAGGAAGTCGCTTCTGTGAAGCAGGCCTTTGATGCGGTAGGGGTGAAATAAAGTGGT
        1930              1950              1970


ATCTCATCAGTGGGGGATTTTTTCCTCCACTGATGTTTTGTTTGTGATCAATGATGTCAG
        1990              2010              2030


GGGAGATAGTTT
    2050
```

## Ansprüche

1. Gen kodierend für Thermolysin und die dazugehörige Präprosequenz aus Bacillus thermoproteolyticus rokko mit der in Tabelle 1 aufgeführten Sequenz.

2. Gen kodierend für das native Thermolysin aus Bacillus thermoproteolyticus rokko mit der in Tabelle 1 aufgeführten Sequenz, beginnend mit der Position 1024 und endend mit der Position 1974.

3. Hybridvektor, enthaltend das Gen nach Anspruch 1 oder 2.

4. Stamm der Gattung Bacillus oder E. coli enthaltend einen Hybridvektor nach Anspruch 3.

Patentansprüche für folgenden Vertragsstaat: ES

1. Verfahren zur Gewinnung eines Gens, kodierend für Thermolysin und die dazugehörige Präprosequenz aus Bacillus thermoproteolyticus mit der in Tabelle 1 aufgeführten Sequenz, dadurch gekennzeichnet, daß Gesamt-DNA aus Bacillus thermoproteolyticus rokko isoliert wird, diese mit Restriktionsendonukleasen in Fragmente unterschiedlicher Größe zerteilt wird, die Fragmente durch Hybridisierung mit Oligonukleotiden identifiziert werden und isoliert werden.

**Europäisches
Patentamt**

**EUROPÄISCHER
RECHERCHENBERICHT**

Nummer der Anmeldung

**EP 90 11 6858**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | JOURNAL OF GENERAL MICROBIOLOGY, Band 134, Nr. 7, 1988, Seiten 1883-1892; M. KUBO et al.: "Cloning and nucleotide sequence of the highly thermostable neutral protease gene from Bacillus stearothermophilus" * Ganzes Artikel * | 1-4 | C 12 N 15/57 C 12 N 1/21 // (C 12 N 1/21 C 12 R 1:07 ) (C 12 N 1/21 C 12 R 1:19 ) |
|  | – – – | | |
| D,Y | NATURE, NEW BIOLOGY, Band 238, Nr. 80, 1972, Seiten 35-37, London, GB; K. TITANI et al.: "Amino-acid sequence of thermolysin" * Ganzes Artikel * | 1-4 | |
|  | – – – | | |
| A | EP-A-0 309 879 (U.R. GRACE & CO.-CONN.) * Seite 3, Zeilen 11-31; Seite 8; Beispiel III, Ansprüche * | 1-4 | |
|  | – – – – – | | RECHERCHIERTE SACHGEBIETE (Int. Cl.5) |
|  | | | C 12 N |

Der vorliegende Recherchenbericht wurde für alle Patentansprüche erstellt

| Recherchenort | Abschlußdatum der Recherche | Prüfer |
|---|---|---|
| Den Haag | 17 Dezember 90 | HUBER A. |